# EUROPEAN PATENT SPECIFICATION

(11) **EP 2 279 163 B1**
(45) Date of publication and mention of the grant of the patent: **21.05.2014**
(21) Application number: 09742069.9
(22) Date of filing: 05.05.2009
(51) Int. Cl.: C07C 68/06, C07C 69/96, C07C 29/12, C07C 31/20, C07C 27/00

(54) **PROCESS FOR PREPARING ALKANEDIOL AND DIALKYL CARBONATE**
VERFAHREN ZUR HERSTELLUNG VON ALKANDIOL- UND DIALKYLCARBONAT
PROCÉDÉ DE PRÉPARATION D'UN ALCANÉDIOL ET D'UN CARBONATE DE DIALKYLE

(30) Priority: 06.05.2008 EP 08155698
(43) Date of publication of application: 02.02.2011
(73) Proprietor: Shell Internationale Research Maatschappij B.V., 2596 HR The Hague (NL)
(72) Inventor: ALLAIS, Cyrille Paul, 1031 CM Amsterdam (NL); BOELENS, Minne, 1031 CM Amsterdam (NL); VAN DER HEIDE, Evert, 1031 CM Amsterdam (NL)
(74) Representative: Matthezing, Robert Maarten
(86) International application number: PCT/EP2009/055434
(87) International publication number: WO 2009/135851

(56) References cited:
- WO-A-03/033450
- WO-A-03/089400
- US-A1- 2005 234 258

## Description

The present invention relates to a process for the preparation of an alkanediol and a dialkyl carbonate from an alkylene carbonate and an alkanol.

Such transesterification processes are known. According to these known transesterification processes, the reaction of the alkanol with the alkylene carbonate has to be effected in the presence of a transesterification catalyst. See e.g. US-A 5,359,118. This document discloses a process in which di(C₁-C₄ alkyl) carbonates are prepared by transesterification of an alkylene carbonate with a C₁-C₄ alkanol. Thereto, the alkylene carbonate and the alkanol are reacted in the presence of a transesterification catalyst. The catalyst is usually homogeneous, although the use of heterogeneous catalysts is also suggested.

WO 2003 033450 also discloses a process where a cyclic carbonate and an aliphatic monohydric alcohol are reacted in the presence of a transesterification catalyst.

It is desirable, in a process for the preparation of an alkanediol and a dialkyl carbonate from an alkylene carbonate and an alkanol, to react the alkanol with the alkylene carbonate in the absence of a transesterification catalyst.

Surprisingly it was found that in a first stage of such process, the alkanol may indeed be reacted with the alkylene carbonate in the absence of a transesterification catalyst, with an attractive conversion and selectivity, such reaction resulting in a mixture comprising hydroxyalkyl alkyl carbonate, unconverted alkanol and unconverted alkylene carbonate. In addition it was found that in a second stage, further conversion of the mixture comprising hydroxyalkyl alkyl carbonate, alkanol and alkylene carbonate in the presence of a transesterification catalyst results in a mixture comprising the alkanediol and the dialkyl carbonate, from which mixture the alkanediol and the dialkyl carbonate may be recovered.

Accordingly, the present invention relates to a process for the preparation of an alkanediol and a dialkyl carbonate comprising:
(a) reacting an alkylene carbonate and an alkanol at a temperature of from 10 to 200 °C, at a pressure of from 5x10⁴ to 5x10⁶ N/m² (0.5 to 50 bar) and in the absence of a transesterification catalyst, to obtain a mixture comprising hydroxyalkyl alkyl carbonate, alkanol and alkylene carbonate;
(b) contacting the mixture comprising hydroxyalkyl alkyl carbonate, alkanol and alkylene carbonate with a transesterification catalyst at a temperature of from 10 to 200 °C and at a pressure of from 5x10⁴ to 5x10⁶ N/m² (0.5 to 50 bar), to obtain a mixture comprising the alkanediol and the dialkyl carbonate; and
(c) recovering the alkanediol and the dialkyl carbonate from the mixture comprising the alkanediol and the dialkyl carbonate.

The preparation of an alkanediol and a dialkyl carbonate from an alkylene carbonate and an alkanol involves a transesterification reaction mechanism comprising two steps. In a first step, the alkylene carbonate reacts with one molecule of the alkanol to yield an intermediate, namely the hydroxyalkyl alkyl carbonate. In a second step, another molecule of the alkanol reacts with said hydroxyalkyl alkyl carbonate to yield the desired dialkyl carbonate and alkanediol.

It has now been found that said intermediate hydroxyalkyl alkyl carbonate is readily obtained in the absence of a transesterification catalyst. With the absence of a transesterification catalyst in step (a) of the present process, it is meant that in said step (a) the amount of transesterification catalyst is at most 100 parts per million by weight (ppmw), based on alkylene carbonate. Preferably, said maximum amount of transesterification catalyst is 50 ppmw, more preferably 10 ppmw, and even more preferably 1 ppmw. Most preferably, no detectable transesterification catalyst is present in the alkylene carbonate and/or alkanol. Further, preferably, with the absence of a transesterification catalyst in step (a) of the present process, it is meant that in said step (a) no transesterification catalyst is added to the alkylene carbonate and/or alkanol.

In addition to said intermediate hydroxyalkyl alkyl carbonate, some quantities of the alkanediol and dialkyl carbonate products may be formed in the absence of a transesterification catalyst. Therefore, an advantage of the present process it that it is partially carried out in the absence of a catalyst. An advantage of not using a transesterification catalyst, is that less or no by-products are formed. This is demonstrated in the Examples below wherein step (a) of the process of the present invention is further illustrated.

Examples of by-products which, in general, may be formed when a transesterification catalyst is present, are oligomers of the alkanediol, e.g. diethylene glycol (DEG) and triethylene glycol (TEG) in a case where the alkylene carbonate is ethylene carbonate, or dipropylene glycol (DPG) and tripropylene glycol (TPG) in a case where the alkylene carbonate is propylene carbonate. Further examples of such by-products are ether by-products, e.g. 2-ethoxyethanol (oxitol) in a case where the alkylene carbonate is ethylene carbonate and the alkanol is ethanol, or 1-ethoxypropan-2-ol and 2-ethoxypropan-1-ol in a case where the alkylene carbonate is propylene carbonate and the alkanol is ethanol.

Steps (a) and (b) of the present process may be carried out in the same reactor. In such a case, the transesterification catalyst required for step (b) is only added to the reaction mixture at a time at which a certain conversion of the alkylene carbonate into the hydroxyalkyl alkyl carbonate has been achieved.

However, preferably, said steps (a) and (b) are carried out in two different reactors arranged in series. In the first reactor, the alkylene carbonate and the alkanol are reacted in the absence of a transesterification catalyst, to obtain a mixture comprising hydroxyalkyl alkyl carbonate, alkanol and alkylene carbonate. The mixture comprising hydroxyalkyl alkyl carbonate, alkanol and alkylene carbonate is sent, suitably via a piping, from said first reactor to said second reactor. Further, in said second reactor, a transesterification catalyst is provided. By contacting, in said second reactor, the mixture comprising hydroxyalkyl alkyl carbonate, alkanol and alkylene carbonate with the transesterification catalyst, further conversion takes place to obtain a mixture comprising the alkanediol and the dialkyl carbonate.

Advantageously, such process, wherein said steps (a) and (b) are carried out in two different reactors arranged in series, may be carried out continuously.

Further, an advantage of starting the transesterification reaction in a first reactor before feeding to a second reactor containing transesterification catalyst, said reactors being arranged in series, is that at a given catalyst loading and residence time in said second reactor, a higher conversion can be obtained in the second reactor. Further, in a case where said second reactor already runs at equilibrium conversion, an advantage of starting the transesterification reaction in the first reactor, is that the size of and/or the catalyst loading in said second reactor may be reduced. Further advantages relate to temperature control in the second reactor as is explained below.

In cases where it is preferred to be able to maintain a constant temperature in an entire reactor, for example in a plug flow reactor, the formation of areas where the temperature is higher or lower than the set or average temperature for the entire reactor, should be prevented. In the case of the two-step transesterification reaction of an alkylene carbonate with an alkanol, producing firstly the intermediate hydroxyalkyl alkyl carbonate and secondly the dialkyl carbonate and alkylene glycol, said consecutive transesterification reactions may have different thermodynamic behaviors (exothermic, thermodynamically neutral or endothermic). In a process wherein said consecutive transesterification reactions are performed in the same reactor, for example in a plug flow reactor, a difference in thermodynamic behavior between the two reactions could lead to the formation of areas where the temperature is higher or lower than the set or average temperature for the entire reactor. However, in a case where the first transesterification reaction is performed partly or entirely in a separate first reactor, and the second transesterification reaction is performed partly or entirely in a separate second reactor, as is possible in the process of the present invention, temperature control in the second reactor can be carried out in a way such that areas where the temperature is higher or lower than the set or average temperature for the entire reactor, are partly or totally eliminated.

Said first reactor may be a vessel provided with a mixing means, wherein the alkylene carbonate and the alkanol are mixed in the absence of a transesterification catalyst, before the mixture is sent to the second reactor containing a transesterification catalyst. The residence time in said first reactor is in the order of 1 minute to 500 hours. The residence time in said second reactor is also in the order of 1 minute to 500 hours.

Said second reactor may be a reactive distillation column, as described in US-A 5,359,118. This would entail that the reaction is carried out counter-currently. The distillation column may contain trays with bubble caps, sieve trays, or Raschig rings. The skilled person will realise that several types of packings of transesterification catalyst and several tray configurations will be possible. Suitable columns have been described in, e.g., Ullmann's Encyclopedia of Industrial Chemistry, 5th ed. Vol. B4, pp 321 ff, 1992. Preferably, the mixture comprising hydroxyalkyl alkyl carbonate, unconverted alkanol and unconverted alkylene carbonate originating from step (a) of the present process, is fed at the middle part of the reactive distillation column.

Additional alkanol and/or alkylene carbonate may be fed into the reactive distillation column. The alkylene carbonate will generally have a higher boiling point than the alkanol. In the case of ethylene and propylene carbonate the atmospheric boiling points are above 240 °C. Therefore, in general, additional alkylene carbonate will be fed at the upper part of the column and additional alkanol will be fed at the lower part of the column. The alkylene carbonate will flow downwardly, and the alkanol will flow upwardly. The unconverted alkylene carbonate, the hydroxyalkyl alkyl carbonate and possibly additional alkylene carbonate react with the alkanol and are thus converted into the alkanediol and the dialkyl carbonate.

Preferably, step (b) of the present process is conducted in a co-current manner. A suitable way to operate is to carry out the reaction in a trickle-flow manner wherein the reactants part in vapour phase and part in liquid phase drip down over a heterogeneous catalyst. A more preferred way to operate steps (a) and (b) of the process of the present invention is in a reactor with only liquids, one reactor without a catalyst (for step (a)) and one reactor with a catalyst (for step (b)), said reactors being arranged in series. A suitable reaction zone of this type is a pipe-type reaction zone wherein the reaction is conducted in a plug flow manner. At least for said step (b), this will enable the reaction to run to virtual completion. A further possibility is to conduct steps (a) and (b) of the process of the present invention in two separate continuously stirred tank reactors (CSTR) arranged in series. In the latter case the effluent from the CSTR used for performing said step (b), is preferably subjected to a post-reaction in a plug flow reactor so that the reaction runs to virtual completion. During said step (b), additional alkanol and/or alkylene carbonate may be fed.

In step (c) of the present process, the alkanediol and the dialkyl carbonate are recovered from the mixture comprising the alkanediol and the dialkyl carbonate that is formed in step (b). In a case where the mixture comprising the alkanediol and the dialkyl carbonate is formed in a reactive distillation column, separation of said two compounds already takes place in said column itself. In general, the dialkyl carbonate leaves the reactive distillation column as part of the top stream and the alkanediol leaves the column as part of the bottom stream. Said top stream and said bottom stream are then subjected to further separation procedures in order to separate the dialkyl carbonate from unconverted alkanol, and to separate the alkanediol from unconverted alkylene carbonate, respectively.

In other cases, where no reactive distillation column is used in step (b) of the present process, the mixture comprising the alkanediol and the dialkyl carbonate has to be subjected to a separate separation procedure. Said separation may be performed in a first distillation column, whereby the stream from the top of said distillation column comprises the dialkyl carbonate and unconverted alkanol and the stream from the bottom of said distillation column comprises the alkanediol and unconverted alkylene carbonate. Suitable distillation conditions in said first distillation column are a pressure from 0.05 to 1.0 bar and a temperature from 40 to 200 °C.

In a second distillation column, the dialkyl carbonate is recovered from said top stream, and, in a third distillation column, the alkanediol is recovered from said bottom stream.

Said distillation in said second distillation column may suitably be achieved at pressures ranging from subatmospheric pressure to superatmospheric pressure. Suitably the pressure may vary from 0.1 to 45 bar. Temperatures may vary in accordance with the pressure selected. The temperature may be from 35 to 300 °C. More preferably, the conditions in said distillation include a pressure ranging from 0.1 to 1.5 bar and a temperature ranging from 35 to 150 °C.

When the dialkyl carbonate and the alkanol form an azeotrope it may be beneficial to use extractive distillation in said second distillation column, using an extractant to facilitate the separation between the dialkyl carbonate and the alkanol. The extractant can be selected from many compounds, in particular alcohols such as phenol or ethers such as anisole. However, it is preferred to employ an alkylene carbonate as extractant. It is most advantageous to obtain the separation in the presence of the alkylene carbonate that is being used as starting material.

The recovered dialkyl carbonate may optionally be further purified. This further purification may comprise a further distillation step or an ion-exchange step, as described in US-A 5,455,368.

Said distillation in said third distillation column may suitably be achieved at a pressure from 0.01 to 0.4 bar and a temperature of 100 to 200 °C. The top fraction in this distillation containing recovered alkanediol may comprise other compounds, such as unconverted alkylene carbonate depending on the sharpness of the separation cut. Therefore, the recovered alkanediol may optionally be further purified.

The process of the present invention includes the transesterification of an alkylene carbonate with an alkanol. The starting materials of the transesterification are preferably selected from C₂-C₆ alkylene carbonate and C₁-C₄ alkanols. More preferably the starting materials are ethylene carbonate or propylene carbonate and methanol, ethanol or isopropanol, most preferably ethanol.

In step (b) of the present process, the presence of a transesterification catalyst is required. Suitable homogeneous transesterification catalysts have been described in US-A 5,359,118 and include hydrides, oxides, hydroxides, alcoholates, amides, or salts of alkali metals, i.e., lithium, sodium, potassium, rubidium and cesium. Preferred catalysts are hydroxides or alcoholates of potassium or sodium. It is advantageous to use the alcoholate of the alkanol that is being used as feedstock.

Other suitable catalysts are alkali metal salts, such as acetates, propionates, butyrates, or carbonates. Further suitable catalysts are described in US-A 5,359,118 and the references mentioned therein, such as EP-A 274 953, US-A 3,803,201, EP-A 1082, and EP-A 180 387.

As indicated in US-A 5,359,118, it is also possible to employ heterogeneous catalysts. In the current process, the use of heterogeneous transesterification catalysts in step (b) of the transesterification reaction is preferred. Suitable heterogeneous catalysts include ion exchange resins that contain functional groups. Suitable functional groups include tertiary amine groups and quaternary ammonium groups, and also sulphonic acid and carboxylic acid groups. Further suitable catalysts include alkali and alkaline earth silicates. Suitable catalysts have been disclosed in US-A 4,062,884 and US-A 4,691,041. Preferably, the heterogeneous catalyst is selected from ion exchange resins comprising a polystyrene matrix and tertiary amine functional groups. An example is Amberlyst A-21 (ex Rohm & Haas) comprising a polystyrene matrix to which N,N-dimethylamine groups have been attached. Eight classes of transesterification catalysts, including ion exchange resins with tertiary amine and quaternary ammonium groups, are disclosed in J F Knifton et al., J. Mol. Catal, 67 (1991) 389ff.

The transesterification conditions in step (a) and in step (b) of the present process include a temperature of from 10 to 200 °C, and a pressure of from 0.5 to 50 bar (5x10⁴ to 5x10⁶ N/m²). Preferably, especially in co-current operation, said pressure ranges from 1 to 20 bar, more preferably 1.5 to 20 bar, most preferably 2 to 15 bar, and said temperature ranges from 30 to 200 °C, more preferably 40 to 170 °C, most preferably 50 to 150 °C.

Further, preferably an excess of the alkanol over the alkylene carbonate is used in step (a) of the present process. The molar ratio of alkanol to alkylene carbonate in said step (a) is suitably of from 1.01:1 to 25:1, preferably of from 2:1 to 20:1, more preferably of from 4:1 to 17:1, most preferably from 5:1 to 15:1. The amount of catalyst in step (b) of the present process can be of from 0.1 to 5.0 %wt based on alkylene carbonate (i.e. total alkylene carbonate as fed to step (a) of the present process), preferably of from 0.2 to 2 %wt. The weight hourly space velocity in steps (a) and (b) of the present process may suitably range of from 0.1 to 100 kg/kg.hr.

The process of the present invention can be employed for a variety of feedstocks. The process is excellently suited for the preparation of monoethylene glycol (1,2-ethanediol), monopropylene glycol (1,2-propanediol), dimethyl carbonate and/or diethyl carbonate and/or diisopropyl carbonate. The process is most advantageously used for the production of monoethylene glycol or propylene glycol and diethyl carbonate from ethylene carbonate or propylene carbonate and ethanol.

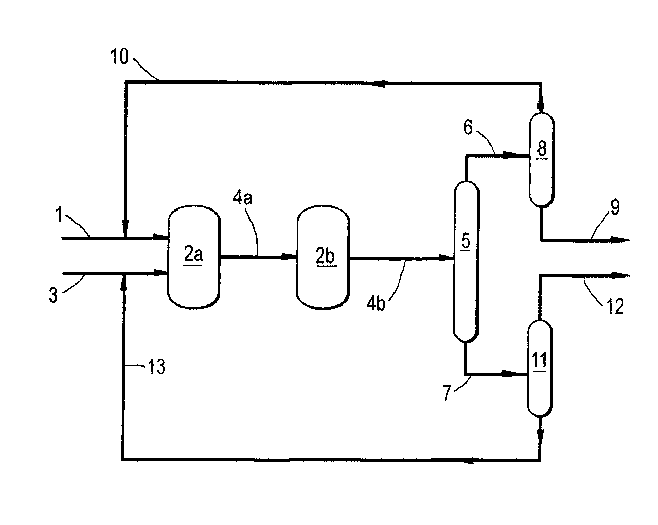

In the figure a flow scheme for the process according to the present invention is shown. Although the process will be described for ethanol as a suitable alcohol and ethylene carbonate as the alkylene carbonate the skilled person will understand that other alkanols and alkylene carbonates can be similarly used.

Ethanol is passed via a line 1 into a reactor 2a. Reactor 2a can suitably be a continuously stirred tank reactor. Reactor 2a does not contain any transesterification catalyst. Via a line 3 ethylene carbonate is also fed into the reactor 2a. Via a line 4a the reaction mixture from reactor 2a, comprising hydroxyethyl ethyl carbonate, ethanol and ethylene carbonate, is fed into reactor 2b. Reactor 2b can also suitably be a continuously stirred tank reactor. A transesterification catalyst is present in reactor 2b, which catalyst may be fed continuously to said reactor. The catalyst may be mixed with the mixture in line 4a or fed to the reactor 2b via a separate line (not shown).

A product comprising a mixture of diethyl carbonate, unconverted ethanol, monoethylene glycol and unconverted ethylene carbonate is withdrawn from the reactor 2b via a line 4b. Via the line 4b the mixture is passed to a distillation column 5 where the product is separated into a top fraction comprising diethyl carbonate and ethanol that is withdrawn via a line 6, and a bottom fraction comprising monoethylene glycol and ethylene carbonate that is withdrawn via a line 7. The mixture comprising diethyl carbonate and ethanol in line 6 is passed to a distillation column 8, where the mixture is separated into ethanol and diethyl carbonate. The diethyl carbonate is discharged via a line 9 and recovered as product, optionally after further purification. Ethanol is recovered via a line 10 and via line 1 recycled to reactor 2a.

The bottom stream in line 7 is subjected to distillation in a distillation column 11. In the distillation column 11 a top product comprising monoethylene glycol is recovered via line 12. Since the top product may be slightly contaminated with some ethylene carbonate further purification may be considered. The bottom product of distillation column 11 withdrawn via line 13 comprises ethylene carbonate. Said ethylene carbonate in line 13 is recycled, optionally after further purification, to reactor 2a via line 3.

It is envisaged that not using a transesterification catalyst, as in step (a) of the process of the present invention, can also be advantageously applied when the dialkyl carbonate is not produced from an alkanol and a (cyclic) alkylene carbonate but from an alkanol and a (non-cyclic) dialkyl carbonate, diaryl carbonate or alkyl aryl carbonate. For example, in a case where diethyl carbonate is to be produced by reacting ethanol with dimethyl carbonate, the ethanol may be reacted with the dimethyl carbonate in the absence of a transesterification catalyst, in a first stage, resulting in a mixture comprising ethyl methyl carbonate, and then in a second stage all of said ethyl methyl carbonate and any unconverted dimethyl carbonate may be converted into diethyl carbonate.

Step (a) of the process of the present invention, wherein no transesterification catalyst is used, is further illustrated by the following Examples.

### Examples

In these experiments, ethylene carbonate (eC; ex Huntsman; purity = 99.99%) and ethanol (EtOH; ex Merck; purity = 99.9%) were used to produce EtOH:eC mixtures at different molar ratios. These molar EtOH:eC ratios are shown in the table below. The molar amount of eC in the mixture was determined. The mixtures did not contain any transesterification catalyst.

The EtOH:eC mixtures were prepared in capped glass vials and stored in an oven at a temperature of 56 °C and under atmospheric pressure (1 bar), for a period of 86 hours. After removal from the oven, the molar amounts of eC, of the half product hydroxyethyl ethyl carbonate (HEEC) and of the final product diethyl carbonate (DEC) were determined by gas chromatography analysis. From these data, the conversion of eC, selectivity to HEEC, selectivity to DEC and selectivity to a certain dimer (see also below) were calculated, as shown in the table below.

| mixture | EtOH:eC ratio (mole) | conversion of eC (%) (1) | selectivity to HEEC (%) (2) | selectivity to DEC (%) (3) | selectivity to dimer (%) (4) |
|---|---|---|---|---|---|
| 1 | 1.9 | 14 | 93.9 | 2.8 | 3.3 |
| 2 | 3.7 | 22 | 94.1 | 2.8 | 3.1 |
| 3 | 5.6 | 26 | 94.2 | 3.3 | 2.5 |
| 4 | 12.9 | 33 | 90.8 | 7.1 | 2.1 |

| | | | | | |
|---|---|---|---|---|---|
| (1) Conversion of eC: (([eC]ₜ₌₀ - [eC]ₜ₌₈₆)/[eC]ₜ₌₀) *100 (2) Selectivity to HEEC: ([HEEC]ₜ₌₈₆/ ([eC]ₜ₌₀ - [eC]ₜ₌₈₆))*100 (3) Selectivity to DEC: ([DEC]ₜ₌₈₆/ ([eC]ₜ₌₀ - [eC]ₜ₌₈₆)) *100 (4) Selectivity to dimer (see also below): 100-"selectivity to HEEC"-"selectivity to DEC" | | | | | |

From the above table it appears that even though no transesterification catalyst was present, advantageously a relatively large portion of eC reacted with EtOH into HEEC, part of which further reacted with EtOH into DEC and monoethylene glycol (MEG). The higher the EtOH:eC ratio the higher the conversion of eC.

Further, advantageously, it was observed that in addition to HEEC, DEC and MEG, only one other product was formed in small quantities (mentioned in the above table as "dimer"), namely a dimer carbonate having the formula

CH₃CH₂OC(O)OCH₂CH₂OC (O) OCH₂CH₃,

which dimer carbonate is formed by reaction of two HEEC molecules.

By-products which may be formed when a transesterification catalyst is present, such as diethylene glycol (DEG), triethylene glycol (TEG) and oxitol (2-ethoxyethanol), were however not detected at all in the above mixtures. It is believed that in cases where such catalyst is present, the back-reaction of eC into ethylene oxide (EO) and carbon dioxide is also promoted. Subsequent reaction of said EO with EtOH results in oxitol, and reaction of said EO with MEG results in DEG, which DEG may further react with said EO into TEG. Therefore, an advantage of the present invention is that through the absence of catalyst in step (a), less by-products have to be removed from the final desired products.

The formation of the above-mentioned dimer carbonate does not result in a loss of desired product, because in step (b) of the process of the present invention wherein transesterification is carried out in the presence of a catalyst or in subsequent step (c) wherein the mixture from said step (b) is subjected to a work-up procedure which may include distillation in distillation columns, the dimer carbonate can be converted into DEC and MEG or into compounds which, possibly after a recycle, can be converted into DEC and MEG. For example, reaction of the dimer carbonate with 2 molecules of EtOH or MEG results in 2 molecules of DEC or HEEC, respectively, and 1 molecule of MEG. Further, for example, reaction of the dimer carbonate with 1 molecule of EtOH or MEG results in 1 molecule of DEC or HEEC, respectively, 1 molecule of eC and 1 molecule of EtOH. HEEC is an intermediate to DEC and MEG. Alternatively, HEEC may react back into eC and EtOH.

Therefore, the only products formed in the experiments of these Examples wherein step (a) of the process of the present invention was performed in the absence of a catalyst, are the desired alkanediol and dialkyl carbonate and products which can be converted at a later stage into said desired products, either in step (b) of the process of the present invention wherein transesterification is carried out in the presence of a catalyst or in subsequent step (c) wherein the mixture from said step (b) is subjected to a work-up procedure. Consequently, the experiments of these Examples have shown that there is no loss of starting material and desired product in step (a) of the present process.

## Claims

1. Process for the preparation of an alkanediol and a dialkyl carbonate comprising:
(a) reacting an alkylene carbonate and an alkanol at a temperature of from 10 to 200 °C, at a pressure of from 5x10⁴ to 5x10⁶ N/m² and in the absence of a transesterification catalyst, to obtain a mixture comprising hydroxyalkyl alkyl carbonate, alkanol and alkylene carbonate;
(b) contacting the mixture comprising hydroxyalkyl alkyl carbonate, alkanol and alkylene carbonate with a transesterification catalyst at a temperature of from 10 to 200 °C and at a pressure of from 5x10⁴ to 5x10⁶ N/m², to obtain a mixture comprising the alkanediol and the dialkyl carbonate; and
(c) recovering the alkanediol and the dialkyl carbonate from the mixture comprising the alkanediol and the dialkyl carbonate.

2. Process according to claim 1, wherein steps (a) and (b) are carried out in two different reactors arranged in series.

3. Process according to claim 2, which is carried out continuously.

4. Process according to claim 2 or 3, wherein step (a) is carried out in a reactor which is a vessel provided with a mixing means.

5. Process according to any of the preceding claims, wherein the temperature in step (a) is of from 30 to 200 °C.

6. Process according to any of the preceding claims, wherein the molar ratio of alkanol to alkylene carbonate in step (a) is of from 2:1 to 20:1.

7. Process according to any of the preceding claims, wherein the transesterification catalyst in step (b) is a heterogeneous catalyst.

8. Process according to any of the preceding claims, wherein the alkylene carbonate is ethylene carbonate or propylene carbonate and the alkanol is ethanol.

## Patentansprüche

1. Verfahren zur Herstellung eines Alkandiols und eines Dialkylcarbonats, umfassend:
(a) Umsetzten eines Alkylencarbonats und eines Alkanols bei einer Temperatur von 10 bis 200 °C bei einem Druck von 5x10⁴ bis 5x10⁶ N/m² und in Abwesenheit eines Umesterungskatalysators, um eine Hydroxyalkyl-alkylcarbonat, Alkanol und Alkylencarbonat umfassende Mischung zu erhalten;
(b) In-Kontakt-Bringen der Hydroxyalkyl-alkylcarbonat, Alkanol und Alkylencarbonat umfassenden Mischung mit einem Umesterungskatalysator bei einer Temperatur von 10 bis 200 °C und bei einem Druck von 5x10⁴ bis 5x10⁶ N/m², um eine den Alkandiol und das Dialkylcarbonat umfassende Mischung zu erhalten; und
(c) Gewinnen des Alkandiols und des Dialkylcarbonats aus der den Alkandiol und das Dialkylcarbonat umfassenden Mischung.

2. Verfahren nach Anspruch 1, wobei die Schritte (a) und (b) in zwei verschiedenen Reaktoren, die in Reihe angeordnet ist, ausgeführt werden.

3. Verfahren nach Anspruch 2, welches kontinuierlich ausgeführt wird.

4. Verfahren nach Anspruch 2 oder 3, wobei der Schritt (a) in einem Reaktor ausgeführt wird, welcher ein mit einem Mittel zum Mischen ausgestatteter Kessel ist.

5. Verfahren nach einem der vorstehenden Ansprüche, wobei die Temperatur im Schritt (a) von 30 bis 200 °C beträgt.

6. Verfahren nach einem der vorstehenden Ansprüche, wobei das Molverhältnis von Alkanol zu Alkylencarbonat im Schritt (a) von 2:1 bis 20:1 beträgt.

7. Verfahren nach einem der vorstehenden Ansprüche, wobei der Umesterungskatalysator im Schritt (b) ein heterogener Katalysator ist.

8. Verfahren nach einem der vorstehenden Ansprüche, wobei das Alkylencarbonat Ethylencarbonat oder Propylencarbonat ist und der Alkanol Ethanol ist.

## Revendications

1. Procédé de préparation d'un alcanediol et d'un carbonate de dialkyle comprenant les étapes de :
(a) faire réagir un carbonate d'alkylène et un alcanol à une température de 10 à 200°C, à une pression de 5x10⁴ à 5x10⁶ N/m² et en absence d'un catalyseur de transestérification, pour obtenir un mélange comprenant du carbonate d'alkyle hydroxyalkyle, de l'alcanol et du carbonate d'alkylène ;
(b) mettre en contact le mélange comprenant du carbonate d'alkyle hydroxyalkyle, de l'alcanol et du carbonate d'alkylène avec un catalyseur de transestérification à une température de 10 à 200°C, et à une pression de 5x10⁴ à 5x10⁶ N/m², pour obtenir un mélange comprenant l'alcanediol et le carbonate de dialkyle ; et
(c) récupérer l'alcanediol et le carbonate de dialkyle du mélange comprenant l'alcanediol et le carbonate de dialkyle.

2. Procédé selon la revendication 1, dans lequel les étapes (a) et (b) sont effectuées dans deux réacteurs différents disposés en série.

3. Procédé selon la revendication 2, qui est effectué en continu.

4. Procédé selon la revendication 2 ou 3, dans lequel l'étape (a) est effectuée dans un réacteur qui est un récipient muni de moyens de mélange.

5. Procédé selon l'une quelconque des revendications précédentes, dans lequel la température à l'étape (a) est de 30 à 200°C.

6. Procédé selon l'une quelconque des revendications précédentes, dans lequel le rapport molaire de l'alcanol sur le carbonate d'alkylène dans l'étape (a) est de 2 :1 à 20 :1.

7. Procédé selon l'une quelconque des revendications précédentes, dans lequel le catalyseur de transestérification de l'étape (b) est un catalyseur hétérogène.

8. Procédé selon l'une quelconque des revendications précédentes, dans lequel le carbonate d'alkylène est le carbonate d'éthylène ou le carbonate de propylène et l'alcanol est l'éthanol.
